# EUROPÄISCHE PATENTANMELDUNG

(11) **EP 1 681 039 A1**
(43) Veröffentlichungstag der Anmeldung: **19.07.2006**
(21) Anmeldenummer: 05000841.6
(22) Anmeldetag: 17.01.2005
(51) Int. Cl.: A61F 2/44

(54) **Paar von Zwischenwirbelprothesen**

(71) Anmelder: Cervitech, Inc., Rockaway, NJ 07866 (US)
(72) Erfinder: Keller, Arnold, 23863 Kayhude (DE)
(74) Vertreter: Glawe, Delfs, Moll

(57) **Zusammenfassung**

Ein Paar von Zwischenwirbelprothesen, die oberhalb und unterhalb desselben Wirbelkörpers (2) zu implantieren sind und jeweils eine mit diesem Wirbelkörper (2) zu verbindende Abschlußplatte (11, 20) sowie einen dieser Abschlußplatte (11, 20) zugeordneten, mit dem genannten Wirbelkörper (2) ventral zu verbindenden Befestigungsflansch (13, 23) aufweisen. Damit die Befestigungsflanschen (13, 23) auch vor Wirbelkörpern mit geringer Höhe hinreichenden Platz finden, sind sie versetzt zueinander angeordnet. Die Versetzung kann seitlich und/oder in AP-Richtung vorgesehen seien.

## Beschreibung

Mitunter ist es erforderlich, zwei in der Wirbelsäule benachbarte Bandscheiben prothetisch zu ersetzen. Es werden dann zwei Zwischenwirbelprothesen oberhalb und eine unterhalb desselben Wirbelkörpers eingesetzt, von denen jede über eine ihrer beiden Abschlußplatten mit diesem Wirbelkörper verbunden wird.

Wenn man befürchten muß, daß die Abschlußplatten keine hinreichende Stabilität am Wirbelkörper erlangen, bevorzugt man solche Prothesen, die mittels ventraler Flanschen am Wirbelkörper befestigt werden. Wenn der Wirbelkörper eine geringe Höhe hat, wie es in der Halswirbelsäule regelmäßig der Fall ist, können die Flanschen, die zu den oberseitig bzw. unterseitig an demselben Wirbelkörper zu befestigenden Abschlußplatten gehören, an der ventralen Seite des Wirbelkörpers miteinander kollidieren.

Dem will die Erfindung vorbeugen. Sie erreicht dies durch die Mittel der Ansprüche 1 oder 12 sowie vorzugsweise durch die Merkmale der Unteransprüche. Die Erfindung setzt als bekannt voraus, daß ein Paar von Zwischenwirbelprothesen oberhalb und unterhalb desselben Wirbelkörpers zu implantieren ist. Jede dieser zwischenwirbelprothesen besteht aus zwei Abschlußplatten und einem dazwischen angeordneten, gelenkigen Prothesenkern. Die untere Abschlußplatte der oberen Prothese wird oberseitig und die obere Abschlußplatte der unteren Prothese mit der Unterseite des Wirbelkörpers verbunden. Die den beiden Abschlußplatten zugeordneten Befestigungsflanschen liegen auf der ventralen Seite des Wirbelkörpers. Dadurch, daß sie erfindungsgemäß versetzt zueinander angeordnet sind, finden sie hinreichenden Platz.

Wenn jede der beiden Abschlußplatten mit Befestigungsflanschen versehen ist, sind diese zweckmäßigerweise in seitlicher Richtung gegeneinander versetzt. Sie können exzentrisch nach entgegengesetzten Seiten hin verschoben sein. Das hat den Vorteil, daß die Flanschanordnung an den Abschlußplatten identisch ist, was die Herstellung erleichtert. Der entgegengesetzte Versatz kommt dadurch zustande, daß die eine Abschlußplatte gegenüber der anderen um 180 ° verdreht ist. Es ist aber auch eine Anordnung denkbar, bei welcher ein Befestigungsflansch mittig und der andere symmetrisch geteilt außermittig angeordnet ist. In diesen Fällen kann jeder Befestigungsflansch mindestens eine Schraubenbohrung enthalten.

Bei einer anderen Ausführungsform der Erfindung sind die Befestigungsflanschen in AP-Richtung gegeneinander versetzt, wobei vorteilhafterweise ein Befestigungsflansch den anderen überlappt. Der überlappte Befestigungsflansch wird von den anderen festgehalten, so daß nur dieser am Knochen befestigt werden und mit einer Schraubenbohrung versehen werden muß.

Die Befestigungsflanschen liegen in der einfachsten Ausführungsform ventral vor dem betreffenden Wirbelkörper. Statt dessen ist es auch möglich, die Befestigungsflanschen in eine ventrale Vertiefung des Wirbelkörpers einzusenken. Man vermeidet auf diese Weise die Irritation des Ösophagus und anderer Organe, die vor der Halswirbelsäule liegen.

Die Befestigungsflanschen können mit den zugehörigen Abschlußplatten einstückig ausgebildet sein. Es kann sich aber auch um separate Teile handeln.

Alternativ kann vorgesehen seien, daß lediglich ein Befestigungsflansch vorhanden ist, der als gesondertes Teil an der ventralen Seite des Wirbelkörpers befestigt wird und mit seinem oberen Rand die Abschlußplatte der oberen und mit seinem unteren Rand die Abschlußplatte der unteren Prothese sichert.

Die Erfindung wird im folgenden näher unter Bezugnahme auf die Zeichnung erläutert. Darin zeigen:
Fig. 1 einen schematischen Sagittalschnitt durch die implantierte Anordnung,
Fig. 2 eine Ventralansicht einer ersten Ausführungsform,
Fig. 3 einen Schnitt gemäß Linie III-III der Fig. 2,
Fig. 4 die Ventralansicht einer zweiten Ausführungsform,
Fig. 5 eine Schnittansicht gemäß Linie V-V der Fig. 4,
Fig. 6 eine Alternative zur Ausführung gemäß Fig. 5 und
Fig. 7 eine dritte Ausführungsform im Sagittalschnitt.

Die aufeinanderfolgenden Wirbelkörper 1, 2, 3 schließen zwei Zwischenwirbelräume 4 und 5 ein, in denen jeweils eine Zwischenwirbelprothese implantiert ist. Die obere Prothese besteht aus einer oberen Abschlußplatte 10, einer unteren Abschlußplatte 11 und einem Prothesenkern 12, wobei die Unterseite der oberen Abschlußplatte 10 und die Oberseite des Prothesenkerns 12 ein Gelenk bilden. Die untere Prothese besteht aus einer oberen Abschlußplatte 20, einer unteren Abschlußplatte 21 und einem Prothesenkern 22, die ebenso ausgebildet sein können wie die entsprechenden Teile der oberen Prothese. Die untere Abschlußplatte 11 der oberen Prothese ist mit der Oberseite des Wirbelkörpers 2 verbunden. Die obere Abschlußplatte 20 der unteren Prothese ist mit der Unterseite des Wirbelkörpers 2 verbunden.

Den Abschlußplatten 11 und 20 sind ventrale Befestigungsflanschen 13, 23 zugeordnet, die Schraubenlöcher 14, 24 enthalten, die die Verschraubung mit dem Wirbelkörper 2 erlauben. Auch die Abschlußplatten 10, 21 können mit ventralen Befestigungsflanschen 6, 7 versehen seien, die aber im vorliegenden Zusammenhang nicht interessieren.

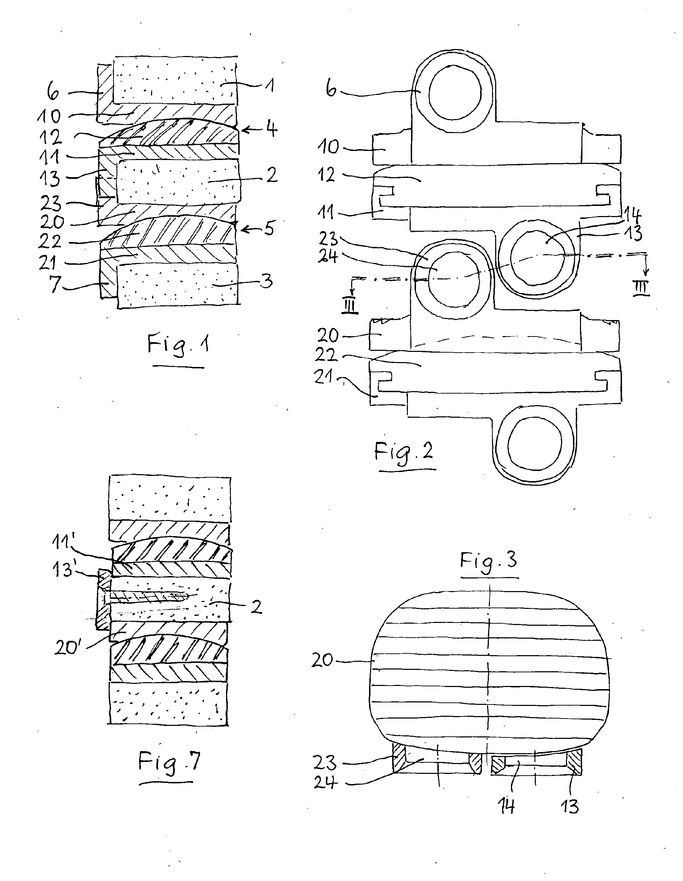

Man erkennt bei Betrachtung der Fig. 1, daß die Befestigungsflanschen 13, 23 nicht übereinander passen, wenn der Wirbelkörper 2 eine geringe Höhe hat, wie es insbesondere bei den Wirbelkörpern der Halswirbelsäule der Fall ist. Sie sind deshalb seitlich versetzt nebeneinander angeordnet, wie Fig. 2 es zeigt. In der dargestellten Ausführungsform sind die Befestigungsflanschen 13, 23 einstückig mit den zugehörigen Abschlußplatten 11, 20 verbunden. Dies muß nicht so sein. Sie können vielmehr gesonderte Teile sein, die mit ihrem freien Rand über die zugehörige Abschlußplatte 11, 20 greifen und sie dadurch im Zwischenwirbelraum sichern.

Während in der Ausführungsform gemäß Fig. 2 und 3 der seitliche Versatz der Befestigungsflanschen zu einer unsymmetrischen Anordnung derselben führt, zeigen Fig. 4 bis 6 eine symmetrische Anordnung. An der oberen Abschlußplatte 11 sind zwei symmetrische Befestigungsflanschen 15 befestigt, die einen Zwischenraum 16 einschließen, in welchem ein mittig angeordneter Befestigungsflansch 25 Platz findet. Wenn dieser mittige Befestigungsflansch 25 schmaler ist als der Zwischenraum 16, sind die Befestigungsflanschen 15, 25 ausschließlich seitlich gegeneinander versetzt. Dargestellt ist in Fig. 4 eine Ausführungsform, in welcher zusätzlich ein Versatz in AP-Richtung vorhanden ist. Die seitlichen Ränder 26 des mittleren Befestigungsflansch 25 greifen hinter die entsprechenden Ränder der Befestigungsflanschen 15. Dadurch wird er von den vor ihm liegenden Befestigungsflanschen 15 am Wirbelkörper gesichert. Er braucht deshalb keine Schraubenbohrung zu enthalten.

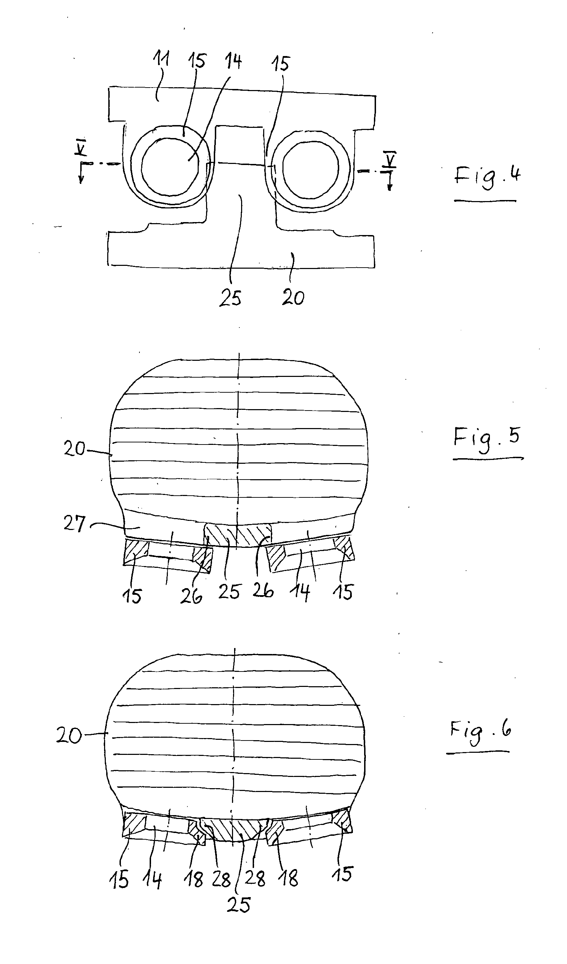

Man erkennt in Fig. 5, daß die Befestigungsflanschen 15, 25 einen nicht unbeträchtlichen Platz in AP-Richtung beanspruchen. Um ihn zu mindern, ist es möglich, den mittleren Befestigungsflansch 25 in der Front des Wirbelkörpers zu versenken. Er wird zu diesem Zweck in die Dimension in AP-Richtung des Teils des Implantats einbezogen, der innerhalb des Zwischenwirbelraums Platz findet. So verfährt man auch mit dem schwach erhöhten Rand 27, der am ventralen Rand der Abschlußplatte 20 angeordneten ist, um ein Hindernis gegen unerwünschte dorsale Verschiebung des Implantats zu bilden.

Eine Variante dieser Ausführung zeigt Fig. 6, bei welcher die Ränder 18, 28 der Befestigungsflanschen 15, 25 derart hinterschnitten sind, daß sie in der gleichen Ebene Platz finden. Die Ränder sind so geformt, daß sie jeweils in den Hinterschnitt des anderen Rands passen. In dieser Form können die Befestigungsflanschen vor dem Wirbelkörper liegen oder in ihm versenkt sein. Im dargestellten Beispiel sind die beiden äußeren Flanschen 15 mit Schraubenlö, chern versehen, während der innere Flansch 25 kein Schraubenloch aufweist, weil er dank des formschlüssigen Ineinandergreifens der Flanschränder 18, 28 von den äußeren Flanschen 15 gehalten wird. Die Anordnung könnte auch umgekehrt getroffen sein, indem die Ränder des innere Flanschs 25 die Ränder der äußeren Flanschen 15 übergreifen und der innere Flansch mit einem Schraubenloch 14 versehen ist.

Fig. 7 veranschaulicht eine andere Lösung desselben Problems. Die zu sichernden Abschlußplatten 11' und 20' sind nicht einstückig mit einem Befestigungsflansch verbunden. Statt dessen ist ein separater Befestigungsflansch 13' vorgesehen, der nach der Implantation der Zwischenwirbelprothesen vor den Wirbelkörper 2 geschraubt ist und dessen Ränder soweit vor die Abschlußplatten 11' und 20' ragen, daß diese nicht nach ventral aus dem Zwischenwirbelraum entweichen können.

## Patentansprüche

1. Paar von zwischenwirbelprothesen, die oberhalb und unterhalb desselben Wirbelkörpers (2) zu implantieren sind und jeweils eine mit diesem Wirbelkörper (2) zu verbindende Abschlußplatte (11, 20) und einen dieser Abschlußplatte (11, 20) zugeordneten, mit dem genannten Wirbelkörper (2) ventral zu verbindenden Befestigungsflansch (13, 23) aufweisen, **dadurch gekennzeichnet, daß** die mit dem genannten Wirbelkörper (2) zu verbindenden Befestigungsflanschen (13, 23) versetzt zueinander angeordnet sind.

2. Paar von Zwischenwirbelprothesen nach Anspruch 1, **dadurch gekennzeichnet, daß** die Befestigungsflanschen (13, 23) in seitlicher Richtung zueinander versetzt sind.

3. Paar von Zwischenwirbelprothesen nach Anspruch 2, **dadurch gekennzeichnet, daß** die Befestigungsflanschen (13, 23) zu entgegengesetzten Seiten hin exzentrisch angeordnet sind.

4. Paar von Zwischenwirbelprothesen nach Anspruch 2, **dadurch gekennzeichnet, daß** der eine Befestigungsflansch (25) mittig und der andere (15) symmetrisch geteilt außermittig angeordnet ist.

5. Paar von Zwischenwirbelprothesen nach Anspruch 1, **dadurch gekennzeichnet, daß** jeder der Befestigungsflanschen (13, 23, 25, 15) mindestens eine Schraubenbohrung (14, 24) enthält.

6. Paar von zwischenwirbelprothesen nach Anspruch 1, **dadurch gekennzeichnet, daß** die Befestigungsflanschen (15, 25) in AP-Richtung gegeneinander versetzt sind.

7. Paar von Zwischenwirbelprothesen nach einem der Ansprüche 1 bis 6, **dadurch gekennzeichnet, daß** ein Befestigungsflansch (15) den anderen (25) überlappt.

8. Paar von zwischenwirbelprothesen nach Anspruch 7, **dadurch gekennzeichnet, daß** nur der ventral überlappende Befestigungsflansch (15) mit wenigstens einer Schraubenbohrung (10) versehen ist.

9. Paar von Zwischenwirbelprothesen nach einem der Ansprüche 1 bis 8, **dadurch gekennzeichnet, daß** wenigstens einer der beiden Befestigungsflanschen (13, 23, 15, 25) zur Einlassung in den Wirbelkörper bemessen ist.

10. Paar von Zwischenwirbelprothesen nach einem der Ansprüche 1 bis 9, **dadurch gekennzeichnet, daß** die Befestigungsflanschen (13, 23, 15, 25) mit der jeweils zugehörigen Abschlußplatte fest verbunden sind.

11. Paar von Zwischenwirbelprothesen nach einem der Ansprüche 1 bis 9, **dadurch gekennzeichnet, daß** die Befestigungsflanschen von der zugehörigen Abschlußplatte gesonderte Teile sind.

12. Paar von Zwischenwirbelprothesen, die oberhalb und unterhalb desselben Wirbelkörpers (2) zu implantieren sind und jeweils eine mit diesem Wirbelkörper (2) zu verbindende Abschlußplatte (11, 20) sowie einen dieser Abschlußplatte (11, 20) zugeordnete, mit dem genannten Wirbelkörper (2) ventral zu verbindenden Befestigungsflansch aufweisen, **dadurch gekennzeichnet, daß** lediglich ein gesonderter Befestigungsflansch (13') vorgesehen ist, der mittels seines oberen und unteren Rands mit beiden Abschlußplatten (11', 20') zusammenwirkt.
